# EUROPEAN PATENT APPLICATION

(11) **EP 3 545 855 A1**
(43) Date of publication of application: **02.10.2019**
(21) Application number: 18170631.8
(22) Date of filing: 03.05.2018
(51) Int. Cl.: A61B 10/00

(54) **KIT FOR OBTAINING A BIOLOGICAL SAMPLE AND METHOD OF IMPROVING PATIENT COMPLIANCE IN THE PROVISION OF SUCH A SAMPLE**

(30) Priority: 26.03.2018 GB 201804842
(71) Applicant: Mast Group Limited, Liverpool, Merseyside L20 1EA (GB); Real Digital International Limited, Dorking, Surrey RH4 2HF (GB)
(72) Inventor: McElarney, Iain, Bootle, Liverpool, Merseyside L20 1EA (GB); Daun, Alexander, Bootle, Liverpool, Merseyside L20 1EA (GB); Stephens, Barry, Dorking, Surrey RH4 2HF (GB); Sargent, Richard, Dorking, Surrey RH4 2HF (GB); Fewings, Steve, Dorking, Surrey RH4 2HF (GB); Monshall, Paul, Dorking, Surrey RH4 2HF (GB); Rivett, Peter, Dorking, Surrey RH4 2HF (GB)
(74) Representative: Rees, Kerry

(57) **Abstract**

The present invention provides a kit of parts for enabling home collection of a biological sample, the kit comprising: an outer packaging box sized to be postable through a standard letter box; an insertion piece comprising a recess for housing a sample collection vessel; a fluid impermeable rigid sample collection vessel suitable for containing a biological sample; a sealable, pre-addressed envelope suitable for housing said sample collection vessel; and a patient advice letter.

## Description

The present invention relates to a kit of parts for enabling the home collection of a biological sample. In particular the present invention relates to a kit of parts for enabling the collection of a clinical or biological sample for the purposes of diagnosis and health screening, such as but not exclusively bowel cancer screening. The invention particularly, but not exclusively, relates to a kit of parts for enabling the collection of a sample for health screening wherein the provision of the kit is unsolicited. The present invention also relates to a method of improving patient compliance in providing biological samples, such as samples suitable for bowel cancer screening.

Biological samples are commonly collected by untrained personnel in non-medical environments. For example, biological samples may be collected by a patient or prospective patient in their own home.

Kits for the home collection of biological samples commonly include a collection tube or vial made from glass or rigid plastic, instructions setting out how the sample should be collected and instructions setting out how the sample may be dispatched to a suitable testing facility.

Kits for the home collection of biological samples are generally dispatched to the user using a postal or courier service.

The provision of kits for enabling the home collection of a biological sample may be solicited or unsolicited. Solicited kits are routinely provided where a user has a concern or interest regarding a particular aspect of their health and/or genealogy. In such cases a user may request, and commonly purchase, a suitable home collection kit for the particular medical concern or interest that they may have. Since the results are of particular interest to the user, compliance levels for completing the sample collection and dispatching for testing are high. However, for unsolicited kits, patient compliance in collecting and returning a suitable biological sample are generally low.

Unsolicited kits for the home collection of biological samples may be provided by regional or national health organisations, charities, research institutions and the like. Kits may commonly be sent out to large populations of people based on age, race, location, sex or other indicia. For example, unsolicited kits may be provided by a regional or national health organisation to all people in a particular population once a particular age is reached.

As disclosed in WO 2014/195694, a biological sample vessel may be provided to a user in a fluid impermeable container configured to meet UN3373-type regulations. Such containers are suitable for providing an empty sample collection vessel and instructions for use to the user. Such containers may also provide a safe means for housing the filled sample vessel and comprise a resealing means in order that the filled sample vessel may be safely dispatched to a suitable testing facility.

In order that the container be suitable for postage, known containers may be made of cardboard coated with a fluid impermeable material to protect postal workers in case of a spillage from the sample collection vessel. The cardboard may also be laminated in order to improve the strength and durability of the container. Such containers may therefore be non-recyclable and costly to produce, deliver and return.

Alternatively, home biological sample collection kits may be provided in an envelope. Such kits may be smaller and lighter than fluid impermeable cardboard container based kits. However, such kits may be more easily lost or damaged during the delivery process. Such kits may also be less appealing to unsolicited users. Recent studies have shown that patient compliance in returning a biological sample following the provision of an envelope based kit is particularly low.

Since unsolicited kits are generally provided at no cost to the user, the cost of producing and delivering such kits is commonly met by a national health organisation, charity or research institution or the like. Unlike solicited kits which are commonly paid for by individual users, large numbers of kits must therefore be paid for by a single organisation. Accordingly, there is a particular need to provide a low cost kit, which may be cheaply delivered, specifically for unsolicited markets, wherein a high level of patient compliance is also achieved.

In order to achieve optimal patient compliance, especially in relation to the use of unsolicited kits, there is a need to provide a kit wherein the collected sample can be easily and cheaply dispatched to a suitable testing facility by the end user or patient, but also has a discreet, professional appearance.

There is therefore a need to provide a kit for home collection of a biological sample wherein the kit is cheap to produce, deliver and return, and has an improved patient compliance rate compared with other known kits, especially when provided unsolicited to a user.

Aspects of the present invention were devised with the foregoing in mind.

According to a first aspect of the present invention there is provided a kit of parts for enabling the home collection of a biological sample. The kit comprises an outer packaging box sized to be postable through a standard letter box; an insertion piece comprising a recess for housing a sample collection vessel; a fluid impermeable rigid sample collection vessel suitable for containing a biological sample; a sealable, pre-addressed envelope suitable for housing said sample collection vessel; and a patient advice letter.

Kits according to the present invention may be suitable for use in enabling the collection of biological samples suitable for testing for any number of medical conditions. Such kits may also be used for the purposes of collecting biological samples for other purposes such as genealogical testing and the like.

It has surprisingly been found that the provision of kit according to the present invention results in substantially higher rates of patient compliance than alternative kits, such as those which comprise outer packaging in the form of an envelope.

Without wishing to be bound by theory, it is considered that kits according to the present invention may be particularly well received for a number of reasons.

Firstly, a kit according to the present invention may have a marked difference in appearance to the majority of post received by a user, which may predominantly be envelope based. Unsolicited post may routinely be thrown away unopened by receivers. However, the difference in appearance from the majority of post received may result in piquing the interest of an unsolicited user, leading to higher numbers of users opening such unsolicited post. Once opened, there is an increased chance of patient compliance in completing the sample provision. In addition, the geometry of a box-based kit may result in less chance of the kit being misplaced than an envelope based kit which may be thinner.

Secondly, the provision of a resealable, pre-addressed envelope suitable for housing the sample collection vessel when filled may provide a discreet way for a patient to dispatch a collected sample to a suitable testing facility. In particular, since it may be that the envelope need only contain the sample vessel, and may not be required to include specimen request sheet or draw an unsolicited user's attention to encourage opening, the envelope may be small enough to fit in a small bag or pocket to allow for discreet transport to a postal collection vessel, such as a post box. Optionally, the envelope may comprise no graphic logos or images suitable for identifying the intended envelope contents or purpose.

The style of envelope may be any suitable style. Preferably, the envelope should be sealable by conventional routes such that it is familiar to a user. In such cases, it may be clear how to insert the test kit and to seal and return the envelope.

The patient advice letter may be a generic letter, for example concerning the biological sample to be collected, the disease or condition being tested for, or the like. Alternatively, the patient advice letter may be personalised and include one or more of the patient's name, address, national health number, GP details, a personalised machine readable code such as a barcode or QR code, and personalised health message targeted to an individual or population demographic

Optionally, the patient advice letter may comprise a specimen request sheet. Alternatively, in some options a specimen request sheet may be included in addition to a patient advice letter. Optionally, additional promotional or marketing materials may also be provided. Such materials may include materials facilitating or promoting general health care initiatives and for that or personalised health messages and information.

The kit may be suitable for enabling the home collection of any biological sample including human tissue, blood, saliva or waste samples. For example, in one embodiment, the kit is configured for enabling the collection of a biological sample suitable for screening for bowel cancer.

Any suitable sample collection vessel may be used. For example an OC-Sensor sample bottle may be used. The sample collection vessel may be provided with a directly traceable code (for example a 1D, 2D or QR) on its surface. The code comprises of a unique patient or kit identifier obtained via any route from a central data source. This may include the software used to manage and operate health screening programs or cancer registries or non-health related databases, for example, such as census or voting registries. The code may or may not include manufacturing lot and expiry information when applied to the sample collection vessel. In this embodiment the participant may be instructed to write the date of sample collection only on the sample vessel although other identifiers can be included for additional security. An advantage of having such traceable code and identifiers on the sample collection vessel is that it minimizes the steps required by the participant to successfully complete the process which increases participation.

The sealable, pre-addressed envelope may be in the form of an envelope upon which an address has been pre-printed. Alternatively, the address may be provided on a label or sticker which has been pre-affixed to the envelope, or provided therewith.

The sealable pre-addressed envelope may be formed of any suitable materials and in some embodiments comprises a fluid impermeable material. Preferably, the envelope is fluid impermeable. For example, the sealable envelope may be formed of plastic.

In some options the envelope may comprise a metallic component. This component may be suitable for triggering sensors, such as sensors in a postal sorting system. This may enable the envelope to be identified as an envelope which should be treated in a particular fashion. For example, this may enable the envelope to be identified as an envelope which should not be entered into particular automated processes which may introduce the envelope to damaging environments, such as overly hot, cold and/or humid environments and/or processes which involve large impact forces, accelerations, an/or turbulence.

According to some embodiments, the envelope may comprise an adsorbent material located within the envelope. Preferably the absorbent material may be fixed within the envelope or integrally formed therewith so as to prevent removal therefrom by the patient. This may ensure that the absorbent material is retained in the envelope when a sample is loaded in the envelope by a user and posted to a suitable testing facility.

In the event of damage, exterior contamination, or incorrect sealing of the sample collection vessel, the absorbent material may aid in preventing the sample from leaking out of the envelope. Optionally, the quantity of absorbent material is sufficient to absorb the entire contents of the sample collection vessel when filled to capacity. Accordingly, the absorbent material may reduce the risk of any potentially hazardous biological material exiting the envelope in error. The absorbent material may be any suitable material, including but not limited to an absorbent foam, such as a super absorbent polymer, a gel or a natural fibre, such as a cellulose matrix or cotton wool. The absorbent material may be an absorbent webbing.

The absorbent material may also have cushioning properties suitable for absorbing forces of impact during transit to prevent damage.

The sealable, pre-addressed envelope may be of any suitable geometry for housing a loaded sample collection vessel and for transport in the outer packaging box. Optionally, the sealable, pre-assembly may be folded during transport in the outer packaging box. Preferably the envelope is small enough so as to be discreetly carried in a bag or pocket prior to return postage to a suitable testing facility. For example, the envelope may have dimensions including a width from 4 to 15 cm, length from 8 to 20 cm, and a depth prior to filling with a sample collection vessel of less than 1 cm. Preferably the envelope may have dimensions of including a width from 8 to 12 cm, such as 10 cm, length from 12 to 16 cm such as 14 cm, and a depth prior to filling with a sample collection vessel of less than 0.5 cm, such as less than 0.1 cm.

Preferably the sealable, pre-addressed envelope may be a prepaid envelope such that no additional postage is required to be paid by the user in order to post the filled envelope to a suitable testing facility. The provision of a prepaid envelope may aid in increasing patient compliance by removing any financial reason not to comply with providing a sample for testing.

Preferably the sealable, pre-addressed envelope may be absent of any indicia which could be used to identify the user, such as a patient name or address. Alternatively, patient identification indicia may be included in the form of a patient number, code, QR code, IQR code, barcode or the like which may enable a patient to be identified by authorised personnel only.

Optionally, the sealable pre-addressed envelope may also contain a unique identifier such as a code number, 2D or QR barcode. This may enable the identification of damaged or contaminated samples upon receipt so that repeat kits can be forwarded to the participant without having to risk exposure to obtain an identifier from the primary sampling device.

The provision of an anonymous or quasi-anonymous return envelope may also yield improved patient compliance compared to kits in which the sample is returning using the same packaging as the kit was initially delivered in and which could potentially still comprise patient identifying information such as an address.

The sealing means provided for by the envelope may be any suitable ceiling means for example double sided adhesive tape, glue, or a moisture and/or heat activated adhesive, may be provided on at least one portion of the envelope and configured to be fixed to a further envelope portion in order to seal the envelope. In some embodiments, the sample collection instructions are positioned on an interior surface of the outer packaging box, such that the instructions are observable by the user upon opening of the outer packaging. The instructions may be adhered to an interior surface of the outer packaging container using any suitable adhering means. In other options, the instructions may be provided in a transparent or translucent envelope which is adhered to an interior surface of the outer packaging container. In some options the instructions may be directly printed on to an interior surface of the outer packaging container. This may prevent the instructions from falling out of the container and/or being misplaced prior to use.

In addition, the provision of instructions on an interior surface of the box may enable the instructions to be freely observed by the user without requiring the instructions to be held. For example, the instructions may be positioned on an interior surface of an openable top flap of the box such that, on opening the box, the top flap may be maintained in an open position by virtue of the stiffness of the box material. As such, the instructions may be positioned in an easily viewed position to be read by a user and maintained in that position without being held by the user. This "hands-free" operation, may enable the user to use both hands to collect the sample, while still being able to clearly view the instructions for collection. The layout of the instructions on an interior surface of the box, combined with the provision of a patient advice that may lead the user through a logical process of understanding what they are being requested to undertake and the steps required to complete the process. This is use for engagement with participants with limited language skills or with visual impairment.

The provision of an outer packaging box sized to be postable through a standard letter box facilitates cheap and effective delivery to the public. Suitable sizes for the outer packaging box include lengths of from 10 to 30 cm, widths of from 10 to 20 cm and depths of from 15 to 3 cm. For example, lengths from 18 to 24 cm, widths from 14 to 18 cm and depths from 1.8 to 2.4 cm. In some embodiments, the outer packaging box has a length of about 21.6 cm a width of about 15.5 cm and a depth of about 2.1 cm. Such dimensions may be large enough to prevent the kit from being easily misplaced, while remaining small enough to fit through letter boxes and to be classed as a large envelope by postal service providers such as the British Royal Mail. As such, a cheaper delivery may be provided for. However, it shall be understood that any suitable dimension or combination of these dimensions may be used.

The insertion piece comprises a recess for housing a collection sample vessel. Optionally, there may be multiple recesses for housing multiple sample devices. The recess or recesses may have any suitable geometry for housing a sample collection vessel, a part thereof, multiple sample collection vessels or parts thereof. Preferably the insertion piece may be configured to prevent undesired movement of the sample collection vessel during transport. For example, the recess or recesses may include a pinch point to prevent undesired movement of a mounted sample collection vessel. This ensures accurate and reproducible presentation when opened by the recipient.

In one embodiment, the recess or recesses comprise a portion having a geometry consistent with that of the sample collection vessel to be housed therein, and one or more cutaway portions or subsidiary recesses in communication therewith, configured to facilitate removal of a sample collection vessel housed in the recess. Optionally, the one or more cutaway portions or subsidiary recesses may be configured to receive a portion of a human finger and/or thumb in order to aid in sample collection vessel removal. Thus, the kit may be usable by a variety of users including those with reduced dexterity and/or grip, such as the elderly or infirm.

Any number of cutaway portions or subsidiary recesses may be provided. Preferably, 1, 2, 3, 4 or 5 cutaway portions or subsidiary recesses are provided. Such recesses may be positioned to align with a natural spacing of human fingers and thumbs.

The kit components may be formed of any suitable material or mixtures therefore. Preferably, some or all of the components are formed of recyclable materials. In some embodiments, the outer packaging box, insertion piece and patient advice letter are each recyclable, biodegradable or digestible.

The outer packaging box may be made of any suitable material, which is preferably a recyclable material. In some embodiments, the outer packaging box comprises cardboard. Cardboard may be easily and cheaply produced, cut and shaped. Thus, the provision of a cardboard outer container may aid in providing a kit which may be mass produced in a cost effective manner, suitable for large scale, unsolicited delivery. A cardboard outer container may also provide suitable strength and durability to survive the rigours of delivery without damage to contents housed therein.

The insertion piece may be made of any suitable material. For example the insertion piece may comprise paper, cardboard, paper mâché, plastic or a mixture thereof. Preferably, the insertion piece may be formed of a recyclable, biodegradable or digestible material. Suitable biodegradable materials may include paper, cardboard, and biodegradable and/or digestible plastics. For example, in one embodiment, the insertion piece comprises a recyclable plastic.

In some embodiments, the insertion piece may be made of a transparent or translucent material. The provision of such an insertion piece may enable additional information printed on an interior surface of the base of the outer packaging box to be visible to a user through the insertion piece when the box is open. Such additional information may include further instructions or information relating to the collection and/or return of a biological sample, information relating to a disease to be screened for, targeted health message, marketing information and/or advertisements.

Alternatively or in addition, the insertion piece may comprise instructions, such as graphical instructions, to guide the user through at least a portion of the test procedure, such as the instructions for loading the filled sample collection vessel in the pre-addressed envelope or returning the envelope. The insertion piece may also comprise moulded or sticker mounted instructions in Braille. Alternatively, all such instructions may be provided on an interior surface of the box.

The insertion piece may be suitable for providing additional strength and resilience to the outer packaging box. This may prevent the contents of the box from being damaged during transit. For example, the insertion piece may be resiliently deformable such that it is capable of absorbing impact forces during transit and/or storage. Alternatively, the insertion piece may be rigid so as to inhibit deformation.

Preferably, the insertion piece may be provided with scalloped edges and/or channelling on one or more of its surfaces. The use of scalloped edges provides structural rigidity to the insertion piece. The use of channelling on the surface of the insertion piece limits deformation of the box and the insertion piece by torque applied by the user. The provision of one or other of these features may enable in the insertion piece to provide increased structural integrity to the box when mounted therein. In addition, such features may support the maintenance of the shape of insertion piece during sample collection vessel mounting in and/or removal from the insertion piece.

In one embodiment, the outer packaging box comprises one or more weakened portions configured to be unsealed by a user to form an opening. The weakened portion may comprise a perforated, scored, split, or layered portion or the like, or any combination thereof.

The weakened portion may exist over a portion of one or more faces. In some options the one or more weakened portions are positioned across two adjacent faces of the outer packaging box. The one or more weakened portions may be configured to allow for an opening to be formed across at least a portion one of the two opposing largest faces of the outer packaging container. The opening may be in in the form of a moveable flap. Preferably, the opening may extend across a majority of such a face so as to provide a large expanse of the outer packaging box which may be opened to provide easy access to the contents contained therein. Accordingly, once opened along the one or more weakened portions, a portion of the outer container may form a movable flap which extends across a majority of one of the two opposing largest faces of the outer packaging container. Optionally, the interior surface such a flap may have sample collection instructions mounted or printed thereon.

In some embodiments, the outer packaging box may comprise a graspable tab in communication with the weakened portion. The graspable tab may be freely graspable by the user. Alternatively, the graspable tab may comprise a perforated portion about one or more of its edges to prevent undesired tab opening during transit.

Preferably, the edge of the tab may be recessed from the bottom edge of the box. This may prevent undesired catching of the tab during transport or storage which could otherwise result in premature and/or undesired opening of the box.

The graspable tab may preferably be positioned on a front surface of the box, and be integrally or connectedly attached to a flap, the edges of which are at least partially defined by the weakened portion. Preferably the flap extends across a substantial portion of the top face of the box, for example the flap extends across at least 50% of the surface area of the top face of the box, preferably at least 70%, at least 80%, or at least 90% of the surface area of the top face of the box. The top face of the box may be understood to be the face of the box which contains the patient name and/or address to which the box has been delivered, having the largest surface area. Alternatively, it shall be understood that the graspable tab and corresponding flap positioned on any suitable faces of the box.

The graspable tab may be provided with one or more cut away portion to aid in user access to the tab. For example, a cut away portion may be provided on either side of the tab. This may provide ease of access, and a safe level access to a user. In particular, such a format may be familiar to a user and aid those with comprehension or dexterity issues.

Preferably, the patient advice letter may be provided above the insertion piece such that upon opening the outer box via the graspable tab and flap, the patient advice letter may be positioned on the open face of the box and may be the first item to be viewed by the user. This may improve patient understanding of the kit and thus result in improved patient compliance. The edges of the patient advice letter are held in place between the insertion piece and the inside face of the card box. The shoulders of the box, defined by the weakened zones to enable the top face of the box to open, are designed to ensure the patient advice letter is gripped to prevent loss of contents in the event of both deliberate and accidental.

According to a second aspect of the present invention, there is provided a method of improving patient compliance in providing a bowel cancer sample; the method comprising providing a kit according to Claim 2.

In one embodiment, the provision of the kit is unsolicited. By unsolicited it is meant that the kit was not directly requested by the end user. Alternatively, the provision of the kit may be solicited.

One or more embodiments of the invention will now be described with reference to the accompanying figures, in which:
Figure 1 is a perspective view of the outer packaging box component of the kit of an embodiment of the present invention.
Figure 2 is a perspective view of the outer packaging box of Figure 1 after opening and removal of the sealable, pre-addressed envelope and patient advice letter.
Figure 3 is a plan view of the rear of a sealable, pre-addressed envelope component of the kit of an embodiment of the present invention.
Figure 4a is a perspective view of an insertion piece according to an embodiment of the present invention.
Figure 4b is a perspective view of an insertion piece according to Figure 4a, having a sample collection vessel mounted therein.

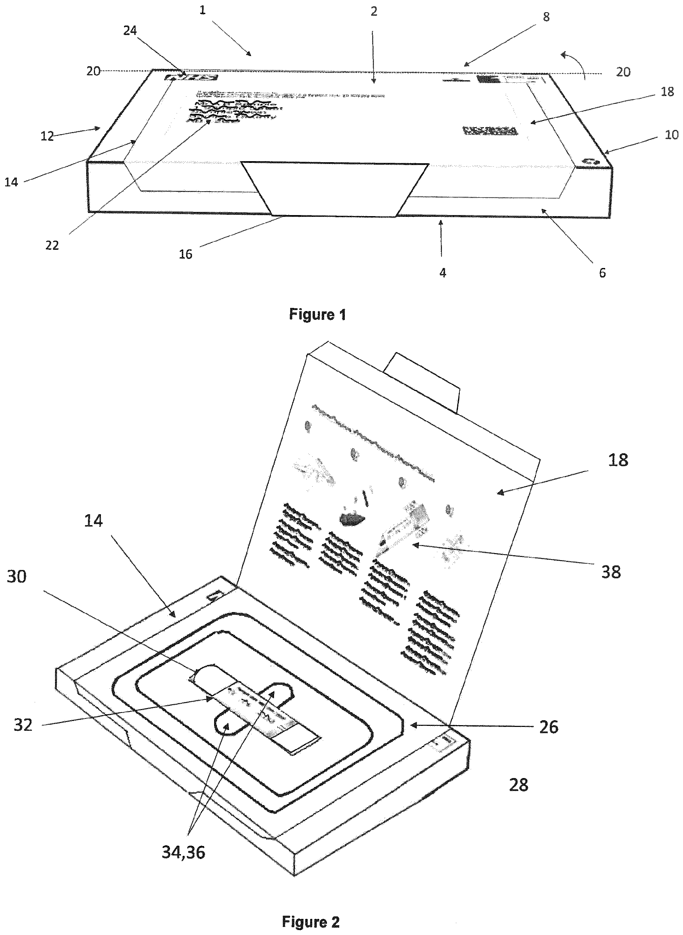

Referring to Figure 1, the kit comprises an outer packaging box 1 sized to be postable through a standard letter box. The outer packaging box is formed from a sheet of cardboard and has a generally cuboidal shape, comprised of a top face 2, opposing bottom face 4, front face 6, opposing rear face 8 and side faces 10 and 12. As shown, the top and bottom faces form the largest planar surfaces of the box.

The top face 2 and front face 6 comprise a line of weakening 14 in communication with a tab 16. To prevent undesired opening during transport, the tab 16 is collectively joined to the remainder of the box by a series of perforations about a portion of its periphery. The line of weakening 14 and tab 16 define a flap 18, rotatable about an axis 20 to form an opening. The flap 18 extends over the majority of the top face to provide easy access to the contents of the box when the flap is rotated to an open position, as shown in Figure 2.

It shall be understood that alternative flap geometries, sizes and positions may also be envisaged.

The outwardly facing surface of the flap 18 comprises a portion 22 for fixing an address label or for printing an address. Additional signs or logos 24 denoting the origin or other information relating to the kit purpose are depicted on the top face of the box. Alternatively, it should be understood that such logos or signs may additionally or alternatively be present on differing faces. Optionally, additional signs or logos may be omitted from the box to provide a more discreet packaging.

Referring to Figure 2, opening the box along the line of weakening (feature 14 in Figure 1) followed by rotation of the resultant flap 18 provides a large opening 26 through which the contents of the box may be accessed. In addition to a sealable, pre-addressed envelope suitable for housing a sample collection vessel and a patient advice letter, not shown, the box comprises an insertion piece 28 and an unfilled sample collection vessel 30, for collecting a biological sample.

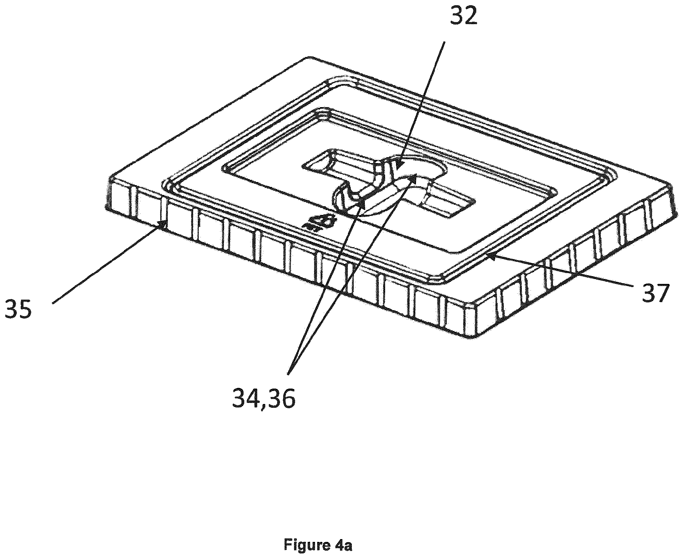

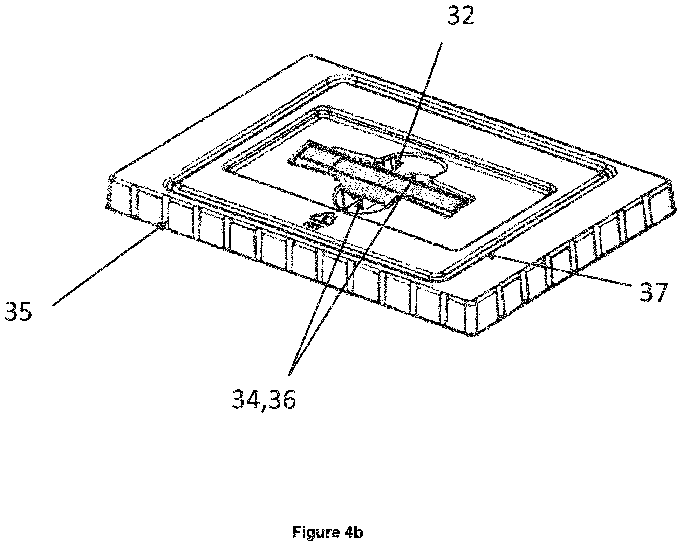

As depicted in Figures 4a and 4b, the insertion piece is formed of a recyclable plastic and has scalloped edges 35. These edges provide additional rigidity to the outer box when the insertion piece is mounted therein. The insertion piece 28 additionally has channeling 37 on the top surface of the inlay.

As depicted in Figure 4b, the sample collection vessel 30 is housed in a recess 32 of the insertion piece 28. The main recess 32 comprises two additional subsidiary recesses 34, 36, suitable for receiving a portion of a human finger and/or thumb in order to grasp a sample collection vessel 30 mounted in the main recess 32. Alternative recess configurations may also be envisioned in which the subsidiary recesses 34 and 36 are altered, omitted and/or relocated relative to the main recess. Additional subsidiary recesses may also be present.

Sample collection instructions 38 are printed on the interior surface of the flap 18 in order that they may be readable by a user when the flap is in the open position depicted in Figure 2. The instructions include both pictorial and written instructions set out as a series of ordered steps to be completed. Alternatively, the instructions may be provided solely in writing. It shall also be understood that the instructions may be provided by way of a label mounted on the flap 18.

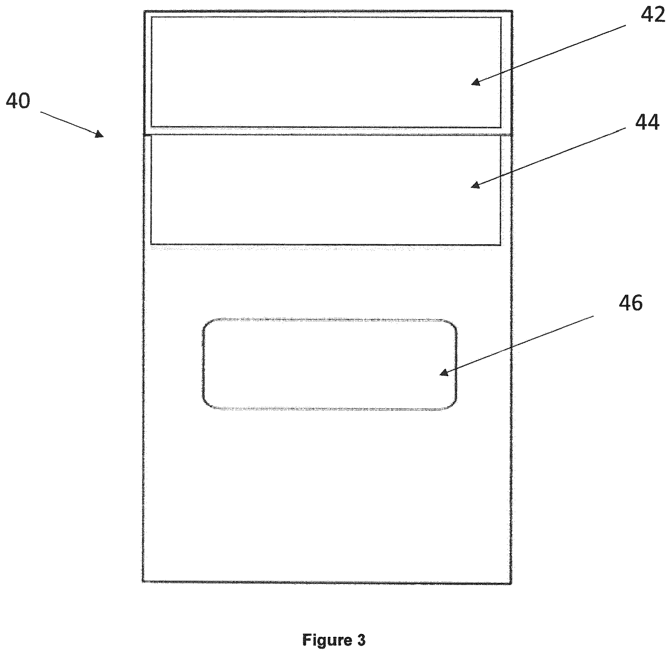

Once collected according to the instructions, the filled sample collection vessel 30 may be loaded in the sealable, pre-addressed envelope 40 according to Figure 3. The envelope 38 comprises sealing portions 42 and 44, at least one of which is provided with an adhesive portion. A removable cover (not shown) may be provided over the adhesive portion of the envelope to prevent premature sealing of the envelope. An instruction box 46 is provided directly below the sealing portions 42 and 44 provide final key instructions to the user. For example the instruction box 46 may contain a reminder for the user to date the sample collection vessel 30 prior to loading in the envelope. Alternatively, the instruction box may be omitted.

The sealable envelope 40 additionally comprises an adsorbent material located inside the sealing envelope, capable of absorbing any biological sample, spillage or leakage from the sample collection vessel 30 once loaded in the envelope 40. The absorbent material is fixed to an interior surface of the envelope to prevent removal therefrom by the user.

In use, and referring to the figures, the sealed outer packaging box may be received by a user through a public postage service or private courier. Most commonly, the kit may be received by the user directly through their own letterbox or the like without the need for collecting the kit from a sorting office or equivalent.

The user grasps the tab 16 and pulls to break the one or more perforations. Continued lifting of the tab in an upward direction with combined rotation about an axis 20 causes the line of weakening 14 to break and the flap 18 to be lifted from the remainder of the outer packaging box 1 to form an opening 26 through which the contents of the packaging box 1 may be accessed. Sample collection instructions 38 positioned on the interior surface of the flap 18 are also exposed.

The user removes the patient advice letter and sealable, pre-addressed envelope 40. The patient advice letter may provide information as to the purpose of the kit. Additional information such as telephone numbers and email addresses where additional information and advice can be found, as well as how to complete the sample collection and or return the sample may also be included.

The user grasps the sample collection vessel 30, mounted in recess 32 of the insertion piece 28, via subsidiary recesses 34 and 36 and pulls to release the sample collection vessel 30 from the packaging. A biological sample may then be collected in accordance with the sample collection instructions 38 and sealed in the sample collection vessel. The date of sample collection may be written on the sample collection vessel by the user. Alternatively, this information may be provided on a separate piece of paper or written on a portion of the sealable envelope 40.

The filled sample collection vessel 30 is then inserted into the sealable, pre-addressed envelope 40. The sealing portions 42 and 44 are then fully sealed, enclosing the sample collection vessel in the envelope 40. For example a protective cover may be removed from sealing portions 42 and/or 44 which comprises an adhesive. The two portions may then be firmly pressed together to seal the envelope 40. Any sample material or liquid that may be present on the exterior of the sample collection vessel, or spilled there from, may be absorbed by an absorbent material which may be provided within the envelope 40.

Once sealed, the user may place the envelope in a post box, or deliver the envelope to a post office or sorting office for delivery to a suitable testing facility.

The use of such a kit may result in higher patient compliance in providing biological samples for testing. In particular, the use of such a kit may result in higher patient compliance in correctly collecting and providing to a suitable testing facility, a sample for bowel cancer screening.

Whilst specific embodiments have been described herein for the purpose of reference and illustration, various modifications will be apparent to a person skilled in the relevant art and may be made without departing from the scope of the invention as defined by the appended claims.

For example, the kit of parts may comprise additional components such as additional information documents, multiple sample collection vessels and the like. The shape, dimensions and materials used to form the outer packaging box, insertion piece or recess thereof, sample collection vessel and sealable, pre-addressed envelope may also be varied.

The kit of the invention provides various communication and user interaction benefits including:
1. The layout of the elements within the box enables the participant to go on a logical journey to understand the process that they are being requested to undertake and the steps required to complete the process.
2. The personalised kit enables the incorporation of specific materials to support specific groups, language and ethnicity requirements.
3. The dual cut opening for the lid provides ease and a safe level of access (the format is familiar and robust to aid those with comprehension or dexterity issues), opening to show introductory copy under the lid, and presenting the formal invitation letter as the first item to view. Graphical instructions to guide the participant through the test process and return procedure will be printed on the bottle-holder.
4. The collection vessel is held in a bottle-holder for consistent presentation but can be easily removed and used. The style of the return envelope is familiar and clear as to how to insert the kit, seal and return in the post.

## Claims

1. A kit of parts for enabling home collection of a biological sample, the kit comprising:
an outer packaging box sized to be postable through a standard letter box;
an insertion piece comprising a recess for housing a sample collection vessel;
a fluid impermeable rigid sample collection vessel suitable for containing a biological sample;
a sealable, pre-addressed envelope suitable for housing said sample collection vessel; and
a patient advice letter.

2. A kit according to Claim 1, wherein the kit is configured for enabling the collection of a biological sample suitable for screening for bowel cancer.

3. A kit according to any preceding claim, wherein sample collection instructions are positioned on an interior surface of the outer packaging box, such that the instructions are observable by the user upon opening of the outer packaging.

4. A kit according to any preceding claim, wherein the outer packaging box has a length of from 10 to 30 cm, a width of from 10 to 20 cm and a depth of from 1.5 to 3 cm.

5. A kit according to any preceding claim, wherein the recess comprises a portion having a geometry consistent with that of the sample collection vessel to be housed therein, and one or more cutaway portions or subsidiary recesses in communication therewith, configured to facilitate removal of a sample collection vessel housed in the recess.

6. A kit according to any preceding claim, wherein the outer packaging box, insertion piece and patient advice letter are each recyclable, biodegradable and/or digestible.

7. A kit according to any preceding claim, wherein the outer packaging box comprises cardboard.

8. A kit according to any preceding claim, wherein the insertion piece comprises a recyclable, biodegradable or digestible plastic.

9. A kit according to any preceding claim, wherein the insertion piece is at least partially formed of a transparent or translucent material.

10. A kit according to any preceding claim, wherein the outer packaging box comprises one or more weakened portions configured to be unsealed by a user to form an opening.

11. A kit according to Claim 10, wherein the outer packaging box further comprises a graspable tab in communication with the weakened portion.

12. A kit according to any preceding claim, wherein the sealable, pre-addressed envelope comprises a fluid impermeable material.

13. A kit according to any preceding claim, wherein the sealable, pre-addressed envelope comprises an adsorbent material located within the envelope.

14. A method of improving patient compliance in providing a bowel cancer sample; the method comprising providing a kit according to Claim 2.

15. A method according to Claim 14, therein the provision of the kit is unsolicited.
